# EUROPEAN PATENT APPLICATION

(11) **EP 4 435 081 A1**
(43) Date of publication of application: **25.09.2024**
(21) Application number: 24163241.3
(22) Date of filing: 13.03.2024
(51) Int. Cl.: C12M 1/00, C12M 3/00, C12M 1/12

(54) **PERMEABLE MEMBRANE SUPPORT**

(30) Priority: 16.03.2023 KR 20230034705
(71) Applicant: ST1 CO., LTD., Busan 46241 (KR)
(72) Inventor: YOON, Yong Jun, 46232 Busan (KR); KO, Byung Jun, 51329 Changwon-si (KR)
(74) Representative: Schmid, Nils T.F.

(57) **Abstract**

A permeable membrane support having a removable permeable membrane is disclosed The permeable membrane support includes a lower support having a hollow cylinder shape with an outer diameter smaller than an inner diameter of a plate well so to be accommodated in the plate well, wherein the lower support includes: an upper end having a first opening and a lower end having a second opening; an inner wall extending between the upper and lower ends and having an open channel defined therein and extending therebetween and communicating with the first and the second openings; a support surface portion extending inwardly from an inner surface of a lower end of the inner wall and having a central opening defined therein constituting the open channel; an upper support including: a cylindrical lower member constructed to be partially accommodated in the lower support; a pair of opposing wings constructed to be supported on a top of the plate well; and a deformable beam portion extending upwardly from an upper circumferential end of the cylindrical lower member to the two opposing wings so as to connect the two opposing wings and the cylindrical lower member to each other; and a permeable membrane disposed between the support surface portion of the lower support and a bottom surface of the cylindrical lower member of the upper support, wherein the permeable membrane includes a porous thin-film and a sealing pattern bonded to a circumference of the porous thin-film, wherein the inner wall of the lower support and the deformable beam portion of the upper support include a hook and a groove, respectively, or include a groove and a hook, respectively, for snap-fit coupling between the lower and upper supports.

## Description

### BACKGROUND

### Field

The present disclosure relates to a permeable membrane support (hereinafter, referred to as PMS) also called a transwell.

### Description of Related Art

A transwell or a permeable membrane support is used for in vitro culture of tissues and organs and evaluation of their functionality in tissue engineering; passing a (potential) drug or toxic substance through a permeable membrane and observing the cell's response for the purpose of drug/toxic substance delivery; research on cell responses to drugs, environment, etc. using cell chemotactic reactions in the field of cell migration & invasion; and attaching different cells to the upper and lower layers of a permeable membrane or cultivating two or more different cells in layers on the upper layer to make it functional in the field of cell co-culture.

For example, different environments in terms of the cell biology are created above and under the permeable membrane, and whether the cells pass through the permeable membrane or not (referred to as cell migration or chemotaxis) is evaluated. Thus, based on the evaluation result, a cell's response to or preference to a target environment is evaluated. In this case, pores of the permeable membrane have a diameter of 3 to 8 µm that is suitable for cells to pass through.

Moreover, in the PMS, cells are cultivated in a culture dish of one of the upper and lower layers around the permeable membrane, while a specific substance, such as drugs and toxins is contained in a culture dish of the other thereof. A reaction of cells is observed when the substance passes through a permeable membrane and is delivered to the cells. In this case, the pore size of the permeable membrane is at a value sized that the cells cannot pass through the membrane. For example, the pore diameter may be smaller than or equal to 3 µm, typically 1 to 0.4 µm.

Recently, hydrogel is gelated on the permeable membrane of the PMS, and cells or drugs are encapsulated in the gel for application research thereof.

In a typical PMS, a permeable thin-film made of PC, PET, or PTFE is attached to a bottom of a transparent support of a PS structure via bonding. As a result, when separating the cultured cells therefrom after culturing various types of cells, deformation is made thereto, and it is difficult to separate the same therefrom into an intact layer.

Moreover, when using the hydrogel, it is difficult to separate the cultured sample therefrom. Generally, a spoon is used to scoop out a portion thereof. When the sample is used without separation thereof from the hydrogel, it is difficult to perform direct measurement using a microscope, etc. due to its size.

Recently, there have been attempts to apply various conditions sequentially or to directly use tissues cultured on PMS for another research. However, in these cases, it is difficult to perform sequential testing in different environments due to the difficulty in the separation thereof from the hydrogel.

It is difficult to directly separate the sample containing cell groups or tissues cultured on the PMS or the hydrogel therefrom or to use the same in various tests, measurements, and experiments in an attached state.

### SUMMARY

A purpose of the present disclosure is to provide a permeable membrane support with a new structure in which a permeable thin-film can be easily separated therefrom.

Purposes according to the present disclosure are not limited to the above-mentioned purpose. Other purposes and advantages according to the present disclosure that are not mentioned may be understood based on following descriptions, and may be more clearly understood based on embodiments according to the present disclosure. Further, it will be easily understood that the purposes and advantages according to the present disclosure may be realized using means shown in the claims and combinations thereof.

A first aspect of the present disclosure provides a permeable membrane support having a removable permeable membrane, the permeable membrane support comprising: a lower support having a hollow cylinder shape with an outer diameter smaller than an inner diameter of a plate well so to be accommodated in the plate well, wherein the lower support includes: an upper end having a first opening and a lower end having a second opening; an inner wall extending between the upper and lower ends and having an open channel defined therein and extending therebetween and communicating with the first and the second openings; a support surface portion extending inwardly from an inner surface of a lower end of the inner wall and having a central opening defined therein constituting the open channel; an upper support including: a cylindrical lower member constructed to be partially accommodated in the lower support; a pair of opposing wings constructed to be supported on a top of the plate well; and a deformable beam portion extending upwardly from an upper circumferential end of the cylindrical lower member to the two opposing wings so as to connect the two opposing wings and the cylindrical lower member to each other; and a permeable membrane disposed between the support surface portion of the lower support and a bottom surface of the cylindrical lower member of the upper support, wherein the permeable membrane includes a porous thin-film and a sealing pattern bonded to a circumference of the porous thin-film, wherein the inner wall of the lower support and the deformable beam portion of the upper support include a hook and a groove, respectively, or include a groove and a hook, respectively, for snap-fit coupling between the lower and upper supports, wherein when the upper support and the lower support have been fastened to each other in a snap-fit manner, the sealing pattern overlaps with and is positioned between the bottom surface of the cylindrical lower member and the support surface portion of the lower support.

In one embodiment of the first aspect, the permeable membrane support further comprises: two or more grooves or protrusions defined in or on an inner surface of the inner wall of the lower support and extending in a length direction thereof; and two or more protrusions or grooves formed on or in an outer surface of the cylindrical lower member of the upper support and extending in a length direction thereof, wherein the two or more grooves or protrusions defined in or on the inner surface of the inner wall of the lower support positionally correspond to the two or more protrusions or grooves formed on or in the outer surface of the cylindrical lower member of the upper support.

A second aspect of the present disclosure provides a permeable membrane support having a removable permeable membrane, the permeable membrane support comprising: a lower support having a hollow cylinder shape with an outer diameter smaller than an inner diameter of a plate well so to be accommodated in the plate well, wherein the lower support includes: an upper end having a first opening and a lower end having a second opening; an inner wall extending between the upper and lower ends and having an open channel defined therein and extending therebetween and communicating with the first and the second openings; a support surface portion extending inwardly from an inner surface of a lower end of the inner wall and having a central opening defined therein constituting the open channel; an upper support including: a cylindrical lower member constructed to be partially accommodated in the lower support; a pair of opposing wings constructed to be supported on a top of the plate well; and a deformable beam portion extending upwardly from an upper circumferential end of the cylindrical lower member to the two opposing wings so as to connect the two opposing wings and the cylindrical lower member to each other; and a permeable membrane disposed between the support surface portion of the lower support and a bottom surface of the cylindrical lower member of the upper support, wherein the permeable membrane includes a porous thin-film and a sealing pattern bonded to a circumference of the porous thin-film, wherein the inner wall of the lower support includes first sawtooth protruding from an inner surface thereof and extending downwardly and outwardly inclinedly, wherein the deformable beam portion of the upper support includes second sawtooth protruding from an outer surface thereof and extending upwardly and outwardly inclinedly, wherein when the upper support and the lower support have been fastened to each other in a snap-fit manner, the sealing pattern overlaps with and is positioned between the bottom surface of the cylindrical lower member and the support surface portion of the lower support.

In one embodiment of the second aspect, the permeable membrane support further comprises: two or more grooves or protrusions defined in or on an inner surface of the inner wall of the lower support and extending in a length direction thereof; and two or more protrusions or grooves formed on or in an outer surface of the cylindrical lower member of the upper support and extending in a length direction thereof, wherein the two or more grooves or protrusions defined in or on the inner surface of the inner wall of the lower support positionally correspond to the two or more protrusions or grooves formed on or in the outer surface of the cylindrical lower member of the upper support.

In one embodiment of the first and/or second aspect, the permeable membrane may be a porous thin-film, for example, a thin-film made of a nanofiber layer, and may include a sealing pattern bonded to the porous thin-film. The sealing pattern may be a circular pattern surrounding a circumference of the porous thin-film. When the upper support and the lower support are fastened with each other in the snap-fit manner, the sealing pattern overlaps and is disposed between the bottom opening of the cylindrical lower member and the support surface portion of the lower support to serve as a seal, that is, to allow cells, drugs, chemicals, metabolites, metabolic wastes, particles, etc. to pass through only the permeable membrane. Depending on the pore size of the permeable membrane, the type of the substance that passes through the permeable membrane may be determined.

The porous thin-film may be a thin-film of a nanofiber layer, and the nanofiber layer may include a polymer nanofiber. The nanofiber layer may be made of a non-woven fiber mat in which nanofibers are oriented irregularly, or a directional fiber mat in which nanofibers are oriented in one direction. Moreover, the nanofiber layer may be made of a woven fiber mat in which the nanofibers are oriented only in two directions that intersect each other.

The sealing pattern may be joined to the porous thin-film. In this regard, "being joined" is defined as a state in which two different components are physically/mechanically joined together without a separate adhesive member. The sealing pattern may be joined thereto via fused deposition modeling. There is no limit to a material of the sealing pattern. The sealing pattern may be made of a material that may seal the lower support and the upper support, such as silicone or urethane.

By using the permeable membrane support according to the present disclosure, the permeable membrane may be independently and safely removed. Thus, when separating cultured cells therefrom after culturing various types of cells, the permeable membrane may be separated therefrom as the an intact layer without deformation of the permeable membrane, and the removed permeable membrane may be placed on the stage of the microscope and then the cultured samples on the permeable membrane may be directly measured using the microscope.

Moreover, due to the snap-fit coupling structure between the upper support and the lower support, the coupling and decoupling therebetween may be easy. The coupling and separation between the upper support and the lower support may be easily accomplished.

Moreover, since the vertical dimension of the permeable membrane support may be adjusted, the upper and lower supports may be stably fastened to each other even when using a thick permeable membrane or a permeable membrane of multiple layers.

In addition to the effects as described above, specific effects in accordance with the present disclosure will be described together with following detailed descriptions for carrying out the disclosure.

### BRIEF DESCRIPTION OF DRAWINGS

FIG. 1 is an exploded perspective view of a permeable membrane support according to a first or third embodiment of the present disclosure.
FIGS. 2 to 4 are diagrams for illustrating a lower support, an upper support, and a permeable membrane of the permeable membrane support according to the first or third embodiment of the present disclosure, respectively.
FIG. 5 is a diagram for illustrating assembly and separation of the permeable membrane support according to the first embodiment of the present disclosure.
FIGS. 6 and 7 are diagrams showing a combined state of the permeable membrane support according to the first or third embodiment of the present disclosure.
FIG. 8 is a diagram for illustrating a permeable membrane support according to a second embodiment of the present disclosure.
FIGS. 9 to 10 are diagrams for illustrating the permeable membrane support according to the third embodiment of the present disclosure.

### DETAILED DESCRIPTIONS

Advantages and features of the present disclosure, and a method of achieving the advantages and features will become apparent with reference to embodiments described later in detail together with the accompanying drawings. However, an embodiment of embodiments of the present disclosure are not limited to the embodiments as disclosed under, but may be implemented in various different forms. Thus, these embodiments are set forth only to make the present disclosure complete, and to entirely inform the scope of the present disclosure to those of ordinary skill in the technical field to which the present disclosure belongs, and the present disclosure is only defined by the scope of the claims.

For simplicity and clarity of illustration, elements in the drawings are not necessarily drawn to scale. The same reference numbers in different drawings represent the same or similar elements, and as such perform similar functionality. Further, descriptions and details of well-known steps and elements are omitted for simplicity of the description. Furthermore, in the following detailed description of the present disclosure, numerous specific details are set forth in order to provide a thorough understanding of the present disclosure. However, it will be understood that the present disclosure may be practiced without these specific details. In other instances, well-known methods, procedures, components, and circuits have not been described in detail so as not to unnecessarily obscure aspects of the present disclosure. Examples of various embodiments are illustrated and described further below. It will be understood that the description herein is not intended to limit the claims to the specific embodiments described. On the contrary, it is intended to cover alternatives, modifications, and equivalents as may be included within the spirit and scope of the present disclosure as defined by the appended claims.

A shape, a size, a ratio, an angle, a number, etc. disclosed in the drawings for illustrating embodiments of the present disclosure are illustrative, and an embodiment of embodiments of the present disclosure are not limited thereto.

The terminology used herein is directed to the purpose of describing particular embodiments only and is not intended to be limiting of the present disclosure. As used herein, the singular constitutes "a" and "an" are intended to include the plural constitutes as well, unless the context clearly indicates otherwise. It will be further understood that the terms "comprise", "comprising", "include", and "including" when used in this specification, specify the presence of the stated features, integers, operations, elements, and/or components, but do not preclude the presence or addition of one or more other features, integers, operations, elements, components, and/or portions thereof. As used herein, the term "and/or" includes any and all combinations of one or more of associated listed items. Expression such as "at least one of" when preceding a list of elements may modify the entire list of elements and may not modify the individual elements of the list. In interpretation of numerical values, an error or tolerance therein may occur even when there is no explicit description thereof.

It will also be understood that when a first element or layer is referred to as being present "on" a second element or layer, the first element may be disposed directly on the second element or may be disposed indirectly on the second element with a third element or layer being disposed between the first and second elements or layers. It will also be understood that when a first element or layer is referred to as being present "under" a second element or layer, the first element may be disposed directly under the second element or may be disposed indirectly under the second element with a third element or layer being disposed between the first and second elements or layers.

It will be understood that when an element or layer is referred to as being "connected to", or "coupled to" another element or layer, it may be directly connected to or coupled to another element or layer, or one or more intervening elements or layers therebetween may be present. In addition, it will also be understood that when an element or layer is referred to as being "between" two elements or layers, it may be the only element or layer between the two elements or layers, or one or more intervening elements or layers therebetween may also be present.

In addition, it will also be understood that when an element or layer is referred to as being "between" two elements or layers, it may be the only element or layer between the two elements or layers, or one or more intervening elements or layers therebetween may also be present.

Further, as used herein, when a layer, film, region, plate, or the like is disposed "on" or "on a top" of another layer, film, region, plate, or the like, the former may directly contact the latter or still another layer, film, region, plate, or the like may be disposed between the former and the latter. As used herein, when a layer, film, region, plate, or the like is directly disposed "on" or "on a top" of another layer, film, region, plate, or the like, the former directly contacts the latter and still another layer, film, region, plate, or the like is not disposed between the former and the latter. Further, as used herein, when a layer, film, region, plate, or the like is disposed "below" or "under" another layer, film, region, plate, or the like, the former may directly contact the latter or still another layer, film, region, plate, or the like may be disposed between the former and the latter. As used herein, when a layer, film, region, plate, or the like is directly disposed "below" or "under" another layer, film, region, plate, or the like, the former directly contacts the latter and still another layer, film, region, plate, or the like is not disposed between the former and the latter.

In descriptions of temporal relationships, for example, temporal precedent relationships between two events such as "after", "subsequent to", "before", etc., another event may occur therebetween unless "directly after", "directly subsequent" or "directly before" is not indicated.

When a certain embodiment may be implemented differently, a function or an operation specified in a specific block may occur in a different order from an order specified in a flowchart. For example, two blocks in succession may be actually performed substantially concurrently, or the two blocks may be performed in a reverse order depending on a function or operation involved.

It will be understood that, although the terms "first", "second", "third", and so on may be used herein to describe various elements, components, regions, layers and/or sections, these elements, components, regions, layers and/or sections should not be limited by these terms. These terms are used to distinguish one element, component, region, layer or section from another element, component, region, layer or section. Thus, a first element, component, region, layer or section described under could be termed a second element, component, region, layer or section, without departing from the spirit and scope of the present disclosure.

Spatially relative terms, such as "beneath," "Hereinafter," "lower," "under," "above," "upper," and the like, may be used herein for ease of illustration to illustrate one element or feature's relationship to another element or feature as illustrated in the figures. It will be understood that the spatially relative terms are intended to encompass different orientations of the device in use or in operation, in addition to the orientation depicted in the figures. For example, when the device in the drawings may be turned over, elements described as "below" or "beneath" or "under" other elements or features would then be oriented "above" the other elements or features. Thus, the example terms "below" and "under" may encompass both an orientation of above and below. The device may be otherwise oriented, for example, rotated 90 degrees or at other orientations, and the spatially relative descriptors used herein should be interpreted accordingly.

The features of the various embodiments of the present disclosure may be partially or entirely combined with each other, and may be technically associated with each other or operate with each other. The embodiments may be implemented independently of each other and may be implemented together in an association relationship.

In interpreting a numerical value, the value is interpreted as including an error range unless there is no separate explicit description thereof.

Unless otherwise defined, all terms including technical and scientific terms used herein have the same meaning as commonly understood by one of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

### First embodiment

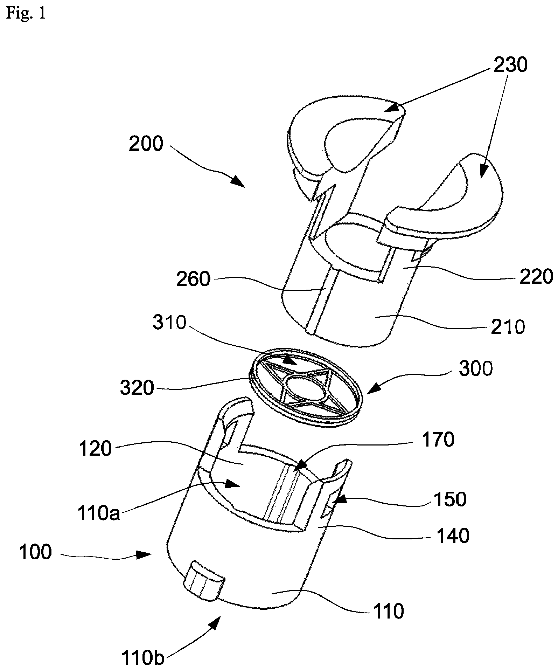

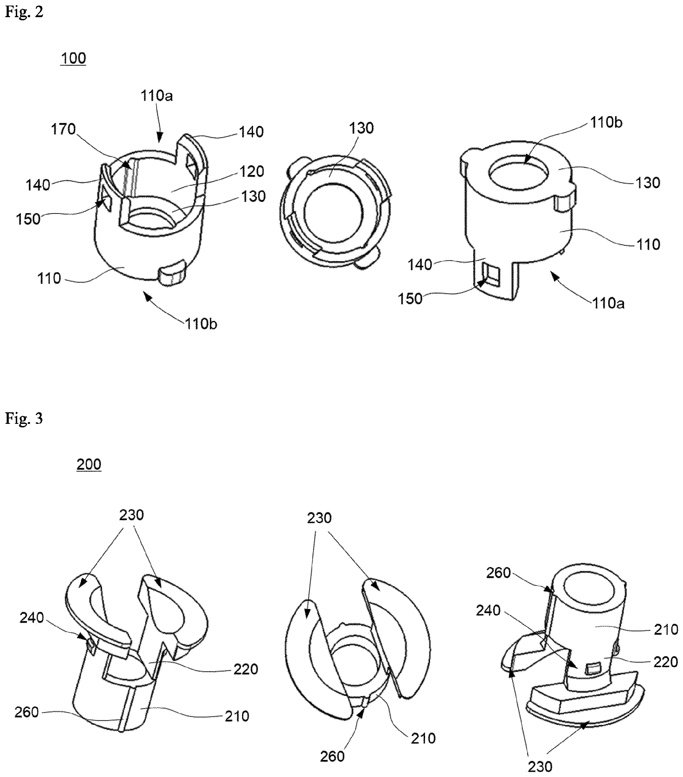

Referring to FIGS. 1 to 4, the permeable membrane support with a detachable permeable membrane according to the first embodiment of the present disclosure includes a lower support 100, an upper support 200, and a permeable membrane 300.

The lower support 100 may be provided in a shape of a hollow cylinder 110 with an outer diameter smaller than an inner diameter of a well of a plate so as to be accommodated in the well of the plate.

The lower support 100 of the hollow cylinder shape may include an upper end with a first opening 110a defined therein and a lower end with a second opening 110b defined therein, and an inner wall 120 extending between the first opening 110a and the second opening 110b, wherein an open channel communicating with the first opening 110a and the second opening 110b is defined in the inner wall 120.

Moreover, the lower support 100 may include a support surface portion 130 that extends inwardly from an inner surface of the lower end of the inner wall 120 and has a central opening defined thereon to constitute the open channel.

The upper support 200 may include a cylindrical lower member 210, a wing 230, and a deformable beam portion 220.

The cylindrical lower member 210 may be a lower portion of the upper support 200, and may be formed to be partially accommodated in the lower support 100. In other words, the cylindrical lower member 210 may be sized so as to be fitted inside the lower support 100.

The wing 230 is formed at a top level of upper support 200 and includes a pair of opposing wings that are stopped and supported on a top of the plate well. The wing 230 is supported on the top of the plate well so that the permeable membrane support may be supported on the top of the plate well. Moreover, the wing 230 may be used as a structure to help facilitate insertion and detachment of the upper support 200 into or from the lower support 100.

The deformable beam portion 220 connects the cylindrical lower member 210 and the wing 230 to each other. Specifically, the deformable beam portion 220 extends vertically upwardly from a circumferential upper end of the cylindrical lower member 210 to the wing 230 and connects the cylindrical lower member 210 and the pair of wings 230 to each other. Therefore, in a top view, an inner circumferential surface of the deformable beam portion 220 has two opposing arc portions and two opposing linear portions, while an outer circumferential surface of the deformable beam portion 220 has a single circle shape. Thus, the deformable beam portion 220 may be inwardly bent more easily than being outwardly bent. Thus, snap-fit coupling of the upper support 200 into the lower support 100 is maintained. The snap-fit coupling may be released by the user bending the deformable beam portion 220 inwardly, allowing for easy removal of the upper support 200 from the lower support 100.

For example, the deformable beam portion 220 may have a relatively thin plate or similar shape so that deflection deformation thereof may be performed. Moreover, the deformable beam portion 220 may be made of an elastic material so that deflection deformation thereof may be performed. Therefore, the deformable beam portion 220 may be easily deformed depending on a direction of an external force. Moreover, the deformable beam portion 220 may have hard physical properties to prevent a spacing between the wings 230 from being increased. Therefore, in absence of the external force, the deformable beam portion 220 may maintain its original shape. That is, the deformable beam portion 220 may be made of a material having physical properties capable of fixing and supporting the wing 230 in the absence of the external force, and having elasticity so that the deformable beam portion 220 may be easily deformed in the direction of the external force in the presence of the external force. The deformable beam portion 220 may be deformed by the user holding and displacing the wings 230. For example, the user may hold the pair of wings 230 and may apply the force thereto such that the spacing between the wings 230 is decreased and thus the deformable beam portion 220 is bent inwardly.

The permeable membrane 300 may be disposed between the lower support 100 and the upper support 200, and may include a porous thin-film 310 and a sealing pattern 320 disposed around the porous thin-film 310.

The porous thin-film 310 may be a thin-film of a nanofiber layer, and the nanofiber layer may include a polymer nanofiber. The nanofiber layer may be made of a non-woven fiber mat in which nanofibers are oriented irregularly, or a directional fiber mat in which nanofibers are oriented in one direction. Moreover, the nanofiber layer may be made of a woven fiber mat in which the nanofibers are oriented only in two directions that intersect each other.

The sealing pattern 320 may be bonded to one or both opposing surfaces of the porous thin-film 310, or may be disposed in contact with a surface of the porous thin-film 310 without being bonded to the porous thin-film 310. Alternatively, the sealing pattern 320 may be in a form that surrounds an edge of the porous thin-film so as to be positioned on one or both opposing surfaces of the edge of the porous thin-film. For example, the sealing pattern 320 may be made of silicone.

This permeable membrane 300 may be disposed between the support surface portion 130 of the lower support 100 and a bottom surface of the cylindrical lower member 210 of the upper support 200. In other words, the permeable membrane may overlap with and be disposed between the support surface portion 130 of the lower support 100 and the bottom surface of the cylindrical lower member 210 when the upper and lower supports are combined with each other in a snap-fit manner. At this time, the permeable membrane covers the opening of the support surface portion 130 of the lower support 100.

In one example, the upper support 200 and the lower support 100 may be combined with each other in a snap-fit manner. To this end, the inner wall 120 of the lower support 100 and the deformable beam portion 220 of the upper support 200 may include a hook (not shown) and a groove (not shown), or a groove 150 and a hook 240, respectively.

In this regard, the lower support 100 may include two opposing protrusions 140 extending upwardly from the upper end with the first opening 110a of the hollow cylinder 110. This protrusion 140 may include a groove 150 or a hook (not shown) corresponding to the hook 240 or a groove (not shown) of the deformable beam portion 220. For example, the protrusion 140 may be made of an elastic material. In the joining process between the lower support 100 and the upper support 200, a spacing between the two opposing protrusions 140 may be slightly increased. However, the two opposing protrusions 140 may return to its original state after the snap-fit fastening.

In one embodiment, the inner wall 120 of the lower support 100 may include the groove 150, while the deformable beam portion 220 of the upper support 200 may include the hook 240. In this case, the hook 240 of the deformable beam portion 220 may protrude outwardly from the upper support 200. A position where the hook 140 and the groove 150 are engaged with each other may be set so that the permeable membrane 300 may overlap with and be disposed between the bottom surface of the cylindrical lower member 210 of the upper support 200 and the support surface portion 130 of the lower support 100. The position may be arbitrarily set by a person skilled in the art depending on a thickness of each of the permeable membrane 300 and the sealing pattern 320.

In another embodiment, the inner wall 120 of the lower support 100 may include a hook (not shown), while the deformable beam portion 220 of the upper support 200 may include a groove (not shown). In this case, the hook (not shown) of the inner wall 120 of the lower support 100 may protrude inwardly from the lower support 100. A structure of this embodiment is not shown in the drawing.

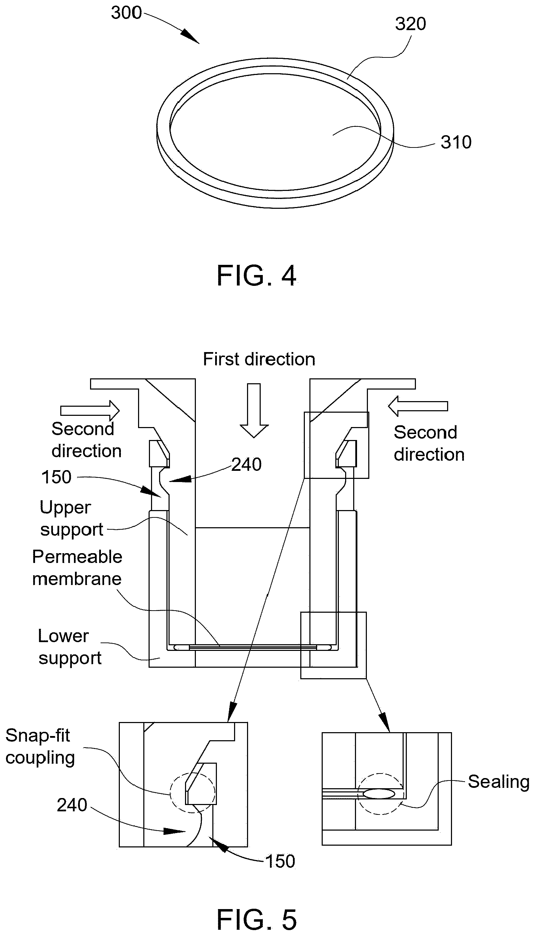

Hereinafter, with reference to FIGS. 5 to 7, a process of assembling and disassembling the lower support 100, the upper support 200, and the permeable membrane 300 of the permeable membrane support according to the first embodiment of the present disclosure is described.

### Assembly process according to first embodiment

First, the permeable membrane 300 is inserted into the lower support 100 through the first opening 110a of the lower support 100 and placed on the support surface portion 130.

Next, the cylindrical lower member 210 of the upper support 200 is inserted into the lower support 100 through the first opening 110a of the lower support 100 in a first direction. In this regard, the cylindrical lower member 210 of the upper support 200 is inserted into the lower support 100 while positioning the two grooves or hooks 240 of the deformable beam portion 220 of the upper support 200 so as to correspond to the two hooks or grooves 150 on the inner surface of the lower support 100, respectively. The deformable beam portion 220 may be deformed in the second direction as it is inserted into the lower support 100. To make this process easier, while the force is applied to the pair of wings 230 in the second direction such that the spacing between the pair of wings 230 of the upper support 200 in the second direction is gradually decreased, the cylindrical lower member 210 of the upper support 200 is inserted into the lower support 100.

When the lower end of the cylindrical lower member 210 of the upper support 200 has contacted the permeable membrane 300, in other words, when the lower end of the cylindrical lower member 210 of the upper support 200 has been inserted to a depth desired by the experimenter, the groove or hook 240 of the deformable beam portion 220 of the upper support 200 may be combined with the hook or groove 150 of the lower support 100 such that the upper support 200 and the lower support 100 are stably assembled with each other in the snap-fit manner.

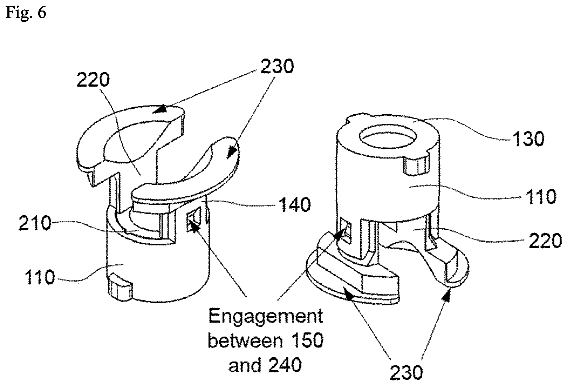

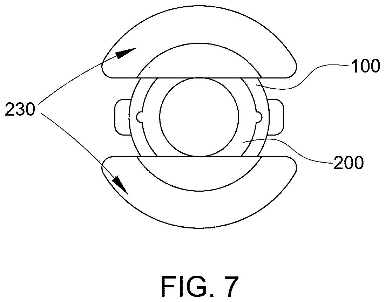

In this way, when the upper support 200 and the lower support 100 are fastened with each other, as shown in FIGS. 6 and 7, the permeable membrane 300 overlaps with and is disposed between the upper surface of the support surface portion 130 of the lower support 100 and the bottom opening of the cylindrical lower member 210 of the upper support 200.

### Separation process according to first embodiment

The separation of the upper support 200 and the lower support 100 from each other may be achieved by first applying the force to the pair of wings 230 of the upper support 200 in the second direction such that the spacing between the pair of wings 230 of the upper support 200 in the second direction is gradually decreased, and thus the two grooves or hooks 240 in the deformable beam portion 220 of the upper support 200 are removed from the two hooks or grooves 150 on the inner surface of the lower support 100, respectively, and then, by pulling the upper support 200 in an opposite direction of the first direction to detach the upper support 200 from the lower support 100, such that the upper support 200 may be easily removed from the inside of the lower support 100.

In this way, only the permeable membrane 300 may be accommodated on the support surface portion 130 of the lower support 100, and then, only the accommodated permeable membrane 300 may be independently removed from within the lower support 100.

### Second embodiment

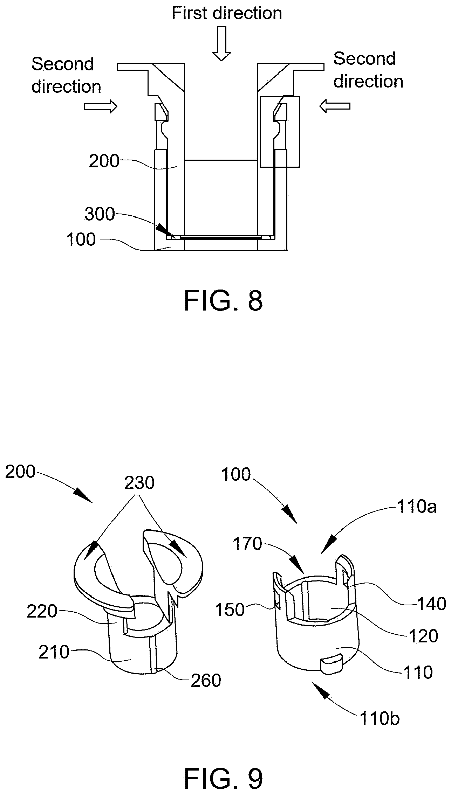

Referring to FIG. 8, the permeable membrane support with the removable permeable membrane according to the second embodiment of the present disclosure includes the lower support 100, the upper support 200, and the permeable membrane 300.

The configuration of the permeable membrane support according to the second embodiment of the present disclosure has the same or similar configuration as or to the permeable membrane support according to the first embodiment except for the groove and hook configuration. Thus, redundant detailed descriptions thereof will be omitted, and following description focuses on differences.

The permeable membrane support according to the second embodiment is different from the permeable membrane support according to the first embodiment in that the upper support 200 and the lower support 100 are coupled to each other in the snap-fit coupling manner using the groove and the hook in the first embodiment, while the upper support 200 and the lower support 100 are coupled to each other in the snap-fit coupling manner using sawtooth (sawtooth threads) 160 and 250.

The inner wall 120 of the lower support 100 includes first sawtooth 160 protruding from the inner surface thereof and extending downwardly and outwardly inclinedly. Each of the first sawtooth 160 includes a first stopper protrusion 160a and a first stopper groove 160b, and has a cross section in an asymmetrical shape.

The deformable beam portion 220 of the upper support 200 includes second sawtooth 250 protruding from the outer surface thereof and extending upwardly and outwardly inclinedly. Each of the second sawtooth 250 includes a second stopper protrusion 250a and a second stopper groove 250b, and a cross section thereof may be formed in an asymmetrical shape so as to be engaged with each of the first sawtooth 160.

The first and second sawtooth 160 and 250 may be engaged with each other and be strongly fastened with each other. Specifically, the second stopper protrusion 250a of each of the second sawtooth 250 is engaged with the first stopper groove 160b of each of the first sawtooth 160, while the first stopper protrusion 160a of each of the first sawtooth 160 is engaged with the second stopper groove 250b of each of the second sawtooth 250. Thus, the first and second sawtooth 160 and 250 may be engaged with each other and be strongly fastened with each other. Even when only a portion of each of the first sawtooth 160 is engaged with each of the second sawtooth 250, a strong fastening force may be achieved. Thus, when permeable membranes of various thicknesses or multi-layered permeable membranes are used, stable fastening between the upper and lower supports may be achieved. Therefore, controlling the engagement of the first and second sawtooth may achieve fastening between the upper and lower supports regardless of the thickness of the permeable membrane used.

In particular, the first and second sawtooth 160 and 250 have cross-sections of the asymmetrical shapes engaged with each other. Through this structure, withdrawal of the cylindrical lower member 210 of the upper support 200 from the lower support 100 may be relatively difficult compared to the insertion of the cylindrical lower member 210 of the upper support 200 in the lower support 100 through the first opening 110a of the lower support 100. In this regard, the withdrawal means that the upper support 200 is removed from the lower support 100, that is, the upper support 200 escapes out from the inside of the lower support 100. That is, because each of the first and second sawtooth 160 and 250 extends in the inclined manner, the withdrawal of the cylindrical lower member 210 of the upper support 200 from the lower support 100 in the opposite direction to the first direction may be relatively difficult compared to the insertion of the cylindrical lower member 210 of the upper support 200 in the lower support 100 through the first opening 110a of the lower support 100 in the first direction.

Hereinafter, with reference to FIG. 8, a process of assembling and disassembling the lower support 100, the upper support 200, and the permeable membrane 300 of the permeable membrane support according to the second embodiment of the present disclosure is described.

### Assembly process according to second embodiment

First, the permeable membrane 300 is inserted into the lower support 100 through the first opening 110a of the lower support 100 and placed on the support surface portion 130.

Next, the cylindrical lower member 210 of the upper support 200 is inserted into the lower support 100 through the first opening 110a of the lower support 100 in a first direction. In this regard, the cylindrical lower member 210 of the upper support 200 is inserted into the lower support 100 such that the first sawtooth 160 and the second sawtooth 250 are engaged with each other. The deformable beam portion 220 may be deformed in the second direction as it is inserted into the lower support 100. To make this process easier, while the force is applied to the pair of wings 230 in the second direction such that the spacing between the pair of wings 230 of the upper support 200 in the second direction is gradually decreased, the cylindrical lower member 210 of the upper support 200 is inserted into the lower support 100.

When the lower end of the cylindrical lower member 210 of the upper support 200 has contacted the permeable membrane 300, in other words, when the lower end of the cylindrical lower member 210 of the upper support 200 has been inserted to a depth desired by the experimenter, the first sawtooth 160 located on the inner surface of the inner wall 120 of the lower support 100 and the second sawtooth 250 located on the outer surface of the deformable beam portion 220 of the upper support 200 are engaged with each other, such that the upper support 200 and the lower support 100 are stably assembled with each other in the snap-fit manner.

As described above, the withdrawal of the cylindrical lower member 210 of the upper support 200 from the lower support 100 in the opposite direction to the first direction may be relatively difficult compared to the insertion of the cylindrical lower member 210 of the upper support 200 in the lower support 100 through the first opening 110a of the lower support 100 in the first direction. Thus, the state in which the upper support 200 and the lower support 100 are assembled with each other in the snap-fit manner may be stably maintained.

In this way, when the upper support 200 and the lower support 100 are fastened with each other, the permeable membrane 300 overlaps with and is disposed between the upper surface of the support surface portion 130 of the lower support 100 and the bottom opening of the cylindrical lower member 210 of the upper support 200.

### Separation process according to second embodiment

The separation of the upper support 200 and the lower support 100 from each other may be achieved by first applying the force to the pair of wings 230 of the upper support 200 in the second direction such that the spacing between the pair of wings 230 of the upper support 200 in the second direction is gradually decreased, and thus the first sawtooth 160 located on the inner surface of the inner wall 120 of the lower support 100 and the second sawtooth 250 located on the outer surface of the deformable beam portion 220 of the upper support 200 are disengaged with and removed from each other and are spaced from each other by a predefined spacing, and then, by pulling the upper support 200 in an opposite direction of the first direction to detach the upper support 200 from the lower support 100, such that the upper support 200 may be easily removed from the inside of the lower support 100. In this regard, the predefined spacing refers to a spacing at which the second stopper protrusion 250a of each of the second sawtooth 250 is not caught in the first stopper groove 160b of each of the first sawtooth 160, that is, each of the second sawtooth 250 can move in the first and/or second direction without being interfered with each of the first sawtooth 160.

In this way, only the permeable membrane 300 may be accommodated on the support surface portion 130 of the lower support 100, and then, only the accommodated permeable membrane 300 may be independently removed from within the lower support 100.

### Third embodiment

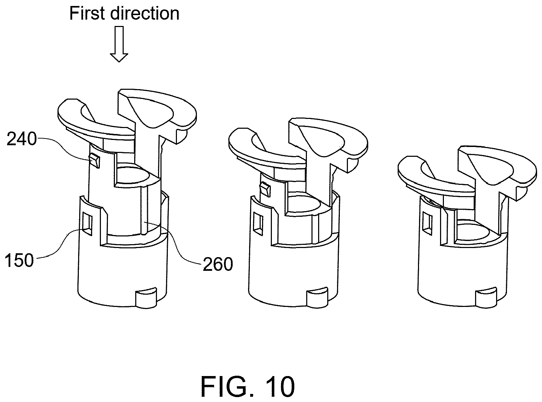

Referring to FIGS. 1 to 4, 6, 7, 9, and 10, the permeable membrane support with the removable permeable membrane according to the third embodiment of the present disclosure includes the lower support 100, the upper support 200, and the permeable membrane 300.

The configuration of the permeable membrane support according to the third embodiment of the present disclosure has the same or similar configuration as or to the permeable membrane support according to the first or second embodiment except for the groove and hook configuration. Thus, redundant detailed descriptions thereof will be omitted, and following description focuses on differences.

The permeable membrane support according to the third embodiment further includes two or more grooves 170 in the inner wall 120 of the lower support 100 and extending in the length direction thereof, and two or more protrusions 260 formed on the cylindrical lower member 210 of the upper support 200 and extending in the length direction thereof for snap-fit coupling. Alternatively, the permeable membrane support according to the third embodiment further includes two or more protrusions (not shown) in the inner wall 120 of the lower support 100 and extending in the length direction thereof, and two or more grooves (not shown) formed on the cylindrical lower member 210 of the upper support 200 and extending in the length direction thereof for snap-fit coupling. This structure may secure further stable combination of the upper support 200 and the lower support 100. These protrusions and grooves may be used as a guide structure for accurate and parallel positioning when the upper support 200 is inserted into the lower support 100 during the snap-fit coupling. Therefore, the assembly and separation between the lower and upper supports 100 and 200 without twisting deformation of the permeable membrane may be achieved.

In a specific example, when the two or more grooves 170 are defined in the inner surface of the inner wall 120 of the lower support 100 and are extending in the length direction thereof, the cylindrical lower member 210 of the upper support 200 includes two or more protrusions 260 formed thereon and arrange in the length direction thereof. Alternatively, when the two or more protrusions (not shown) are formed on the inner surface of the inner wall 120 of the lower support 100 and are extending in the length direction thereof, the cylindrical lower member 210 of the upper support 200 includes two or more grooves (not shown) formed in the outer surface thereof and extending in the length direction thereof.

### Assembly process according to third embodiment

First, the permeable membrane 300 is inserted into the lower support 100 through the first opening 110a of the lower support 100 and placed on the support surface portion 130.

Next, the cylindrical lower member 210 of the upper support 200 is inserted into the lower support 100 through the first opening 110a of the lower support 100 in a first direction. In this regard, the cylindrical lower member 210 of the upper support 200 is positioned relative to the lower support 100 such that the two or more grooves 170 defined in the inner surface of the inner wall 120 of the lower support 100 and extending in the length direction thereof are aligned with and engaged with the two or more protrusions 260 formed on the cylindrical lower member 210 of the upper support 200 and extending in the length direction thereof. Then, the cylindrical lower member 210 of the upper support 200 is inserted into the lower support 100. In this regard, the deformable beam portion 220 may be deformed in the second direction as it is inserted into the lower support 100. To make this process easier, while the force is applied to the pair of wings 230 in the second direction such that the spacing between the pair of wings 230 of the upper support 200 in the second direction is gradually decreased, the cylindrical lower member 210 of the upper support 200 is inserted into the lower support 100.

When the lower end of the cylindrical lower member 210 of the upper support 200 has contacted the permeable membrane 300, in other words, when the lower end of the cylindrical lower member 210 of the upper support 200 has been inserted to a depth desired by the experimenter, the groove or hook 240 of the deformable beam portion 220 of the upper support 200 may be combined with the hook or groove 150 of the lower support 100 such that the upper support 200 and the lower support 100 are stably assembled with each other in the snap-fit manner.

In this way, when the upper support 200 and the lower support 100 are fastened with each other, as shown in FIGS. 6 and 7, the permeable membrane 300 overlaps with and is disposed between the upper surface of the support surface portion 130 of the lower support 100 and the bottom opening of the cylindrical lower member 210 of the upper support 200.

### Separation process according to first embodiment

The separation of the upper support 200 and the lower support 100 from each other may be achieved by first applying the force to the pair of wings 230 of the upper support 200 in the second direction such that the spacing between the pair of wings 230 of the upper support 200 in the second direction is gradually decreased, and thus the two grooves or hooks 240 in the deformable beam portion 220 of the upper support 200 are removed from the two hooks or grooves 150 on the inner surface of the lower support 100, respectively, and then, by pulling the upper support 200 in an opposite direction of the first direction to detach the upper support 200 from the lower support 100, such that the upper support 200 may be easily removed from the inside of the lower support 100.

In this way, only the permeable membrane 300 may be accommodated on the support surface portion 130 of the lower support 100, and then, only the accommodated permeable membrane 300 may be independently removed from within the lower support 100.

Although embodiments of the present disclosure have been described with reference to the accompanying drawings, embodiments of the present disclosure are not limited to the above embodiments, but may be implemented in various different forms. A person skilled in the art may appreciate that the present disclosure may be practiced in other concrete forms without changing the technical spirit or essential characteristics of the present disclosure. Therefore, it should be appreciated that the embodiments as described above is not restrictive but illustrative in all respects.

## Claims

1. A permeable membrane support having a removable permeable membrane, the permeable membrane support comprising:
a lower support having a hollow cylinder shape with an outer diameter smaller than an inner diameter of a plate well so to be accommodated in the plate well, wherein the lower support includes:
an upper end having a first opening and a lower end having a second opening;
an inner wall extending between the upper and lower ends and having an open channel defined therein and extending therebetween and communicating with the first and the second openings;
a support surface portion extending inwardly from an inner surface of a lower end of the inner wall and having a central opening defined therein constituting the open channel;
an upper support including:
a cylindrical lower member constructed to be partially accommodated in the lower support;
a pair of opposing wings constructed to be supported on a top of the plate well; and
a deformable beam portion extending upwardly from an upper circumferential end of the cylindrical lower member to the two opposing wings so as to connect the two opposing wings and the cylindrical lower member to each other; and
a permeable membrane disposed between the support surface portion of the lower support and a bottom surface of the cylindrical lower member of the upper support, wherein the permeable membrane includes a porous thin-film and a sealing pattern bonded to a circumference of the porous thin-film,
wherein the inner wall of the lower support and the deformable beam portion of the upper support include a hook and a groove, respectively, or include a groove and a hook, respectively, for snap-fit coupling between the lower and upper supports,
wherein when the upper support and the lower support have been fastened to each other in a snap-fit manner, the sealing pattern overlaps with and is positioned between the bottom surface of the cylindrical lower member and the support surface portion of the lower support.

2. The permeable membrane support of claim 1, wherein the permeable membrane support further comprises:
two or more grooves or protrusions defined in or on an inner surface of the inner wall of the lower support and extending in a length direction thereof; and
two or more protrusions or grooves formed on or in an outer surface of the cylindrical lower member of the upper support and extending in a length direction thereof,
wherein the two or more grooves or protrusions defined in or on the inner surface of the inner wall of the lower support positionally correspond to the two or more protrusions or grooves formed on or in the outer surface of the cylindrical lower member of the upper support.

3. A permeable membrane support having a removable permeable membrane, in particular according to one of the preceding claims, the permeable membrane support comprising:
a lower support having a hollow cylinder shape with an outer diameter smaller than an inner diameter of a plate well so to be accommodated in the plate well, wherein the lower support includes:
an upper end having a first opening and a lower end having a second opening;
an inner wall extending between the upper and lower ends and having an open channel defined therein and extending therebetween and communicating with the first and the second openings;
a support surface portion extending inwardly from an inner surface of a lower end of the inner wall and having a central opening defined therein constituting the open channel;
an upper support including:
a cylindrical lower member constructed to be partially accommodated in the lower support;
a pair of opposing wings constructed to be supported on a top of the plate well; and
a deformable beam portion extending upwardly from an upper circumferential end of the cylindrical lower member to the two opposing wings so as to connect the two opposing wings and the cylindrical lower member to each other; and
a permeable membrane disposed between the support surface portion of the lower support and a bottom surface of the cylindrical lower member of the upper support, wherein the permeable membrane includes a porous thin-film and a sealing pattern bonded to a circumference of the porous thin-film,
wherein the inner wall of the lower support includes first sawtooth protruding from an inner surface thereof and extending downwardly and outwardly inclinedly,
wherein the deformable beam portion of the upper support includes second sawtooth protruding from an outer surface thereof and extending upwardly and outwardly inclinedly,
wherein when the upper support and the lower support have been fastened to each other in a snap-fit manner, the sealing pattern overlaps with and is positioned between the bottom surface of the cylindrical lower member and the support surface portion of the lower support.

4. The permeable membrane support of any of the preceding claims, wherein the permeable membrane support further comprises:
two or more grooves or protrusions defined in or on an inner surface of the inner wall of the lower support and extending in a length direction thereof; and
two or more protrusions or grooves formed on or in an outer surface of the cylindrical lower member of the upper support and extending in a length direction thereof,
wherein the two or more grooves or protrusions defined in or on the inner surface of the inner wall of the lower support positionally correspond to the two or more protrusions or grooves formed on or in the outer surface of the cylindrical lower member of the upper support.
